(19) Europäisches Patentamt
European Patent Office
Office européen des brevets

(11) Veröffentlichungsnummer: **0 132 541**
**A2**

(12) # EUROPÄISCHE PATENTANMELDUNG

(21) Anmeldenummer: **84106217.7**

(22) Anmeldetag: **30.05.84**

(51) Int. Cl.⁴: **C 07 D 285/10**
C 07 D 295/08, A 61 K 31/445
//C07D209/48, C07D405/06

(30) Priorität: **22.07.83 DE 3326545**

(43) Veröffentlichungstag der Anmeldung:
**13.02.85 Patentblatt 85/7**

(84) Benannte Vertragsstaaten:
**AT BE CH DE FR GB IT LI LU NL SE**

(71) Anmelder: **LUDWIG HEUMANN & CO GMBH**
**Heideloffstrasse 18-28 Postfach 2260**
**D-8500 Nürnberg(DE)**

(72) Erfinder: **Schickaneder, Helmut, Dr. Dipl.-Chem.**
**Moosäcker 25**
**D-8501 Eckental(DE)**

(72) Erfinder: **Postius, Stefan, Dr. Biochem.**
**Unschlittplatz 1**
**D-8500 Nürnberg(DE)**

(72) Erfinder: **Herter, Rolf, Dr. Dipl.-Chem.**
**Bayernstrasse 42**
**D-8540 Schwabach(DE)**

(72) Erfinder: **Mörsdorf, Peter, Dr. Dipl.-Chem.**
**Untere Bahnhofstrasse 16**
**D-8501 Cadolzburg(DE)**

(72) Erfinder: **Szelenyi, Istvan, Dr.**
**Brahmsstrasse 16**
**D-8501 Schwaig(DE)**

(72) Erfinder: **Ahrens, Kurt Henning, Dr.**
**Praterstrasse 9**
**D-8500 Nürnberg(DE)**

(74) Vertreter: **Kraus, Walter, Dr. et al,**
**Patentanwälte Kraus, Weisert & Partner Irmgardstrasse 15**
**D-8000 München 71(DE)**

(54) **Propan-2-ol-Derivate, Verfahren zu ihrer Herstellung und diese Verbindungen enthaltende Arzneimittel.**

(57) Es werden neue Propan-2-ol-Derivate der allgemeinen Formel I:

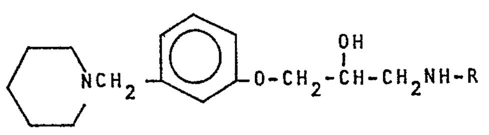

(I)

in der R für

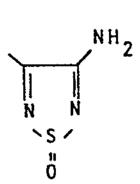

oder

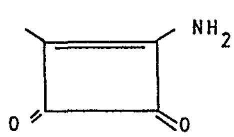

steht, und deren Salze mit pharmakologisch annehmbaren Säuren beschrieben. Diese Verbindungen haben eine selektive Wirkung auf Histamin-$H_2$-Rezeptoren.

EP 0 132 541 A2

0132541

# B E S C H R E I B U N G

Die Erfindung betrifft neue Propan-2-ol-Derivate mit einer hohen selektiven Wirkung auf Histamin-$H_2$-Rezeptoren, Verfahren zu ihrer Herstellung und Arzneimittel, die diese Verbindungen enthalten, sowie schließlich die Verwendung dieser Verbindungen in der Therapie.

Als Antiulcusmittel hat Cimetidin (Tagamet®) bereits Eingang in die Therapie gefunden. Die Halbwertszeit von Cimetidin ist jedoch verhältnismäßig kurz. Dadurch wird es nötig, mehrfach täglich Tabletten mit Dosiseinheiten von 200 bis 300 mg in einer therapeutisch festgelegten Form zu verabreichen. Es besteht somit ein Bedarf an Antiulcusmitteln, die länger wirken und/oder wirksamer sind als Cimetidin.

Die erfindungsgemäßen Verbindungen zeigen aufgrund ihrer spezifischen $H_2$-antagonistischen Aktivität eine Hemmung der Magensäuresekretion, wenn diese durch Histamin-Agonisten stimuliert wird [Ash und Schild, "Brit. J. Pharmacol. Chemother.", 27, 427 (1966) und Black et al., "Nature", 236, 385 (1971)]. Die pharmakologische Aktivität dieser Verbindungen, wie genauer weiter unten beschrieben werden, kann nach einer modifizierten Methode gemäß der DE-OS 27 34 070 beim perfundierten Rattenmagen gezeigt werden. Weiterhin kann die $H_2$-antagonistische Wirkung an wachen Heidenhain-Pouch-Hunden nach der Methode von Black et al., "Nature", 236, 385 (1971) gezeigt werden. Die neuen Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden.

Nachdem Hemmstoffe für Histamin-$H_2$-Rezeptoren sowohl bezüglich der basalen Magensäuresekretion als auch der durch Gastrin, Histamin, Methacholin oder Nahrung induzierten Magensäuresekretion Hemmwirkung besitzen, können sie für die Behandlung von peptischen Ulcera, die durch übermaßige Sekretion von Magensäure verursacht sind, und bei der Therapie hyperacider Gastritis verwendet werden.

Der Erfindung liegt die Aufgabe zugrunde, neue Hemmstoffe für Histamin-$H_2$-Rezeptoren mit verbesserter und/oder länger anhaltender Wirksamkeit zur Verfügung zu stellen.

Diese Aufgabe wird durch die Erfindung gelöst.

Gegenstand der Erfindung sind neue Propan-2-ol-Derivate der allgemeinen Formel I

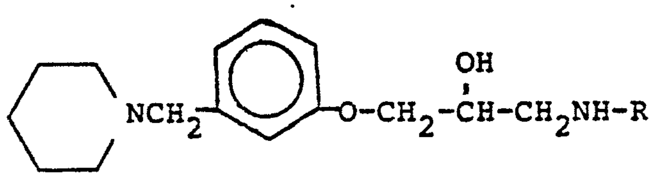

(I)

in der R für

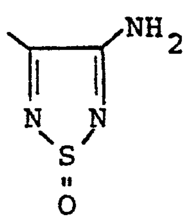

oder

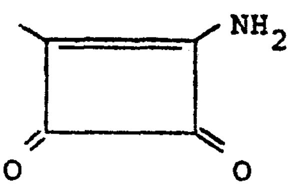

steht, und deren Salze mit pharmakologisch annehmbaren Säuren.

Letztere werden mit geeigneten Säuren gebildet. Beispiele sind die Hydrochloride, Hydrobromide, Sulfate, Methansulfonate, Acetate, Maleate, Succinate, Citrate, Tartrate, Fumarate, Benzoate, Embonate etc. sowie deren Hydrate.

Die Erfindung umschließt weiterhin alle tautomeren Formen und alle optisch aktiven Isomere und deren Salze. Die erfindungsgemäßen Verbindungen der Formel I können Disalze bilden, die gleichfalls unter den Rahmen der vorliegenden Erfindung fallen.

Die erfindungsgemäßen Verbindungen werden durch ein Verfahren hergestellt, das dadurch gekennzeichnet ist, daß man eine Verbindung der Formel II

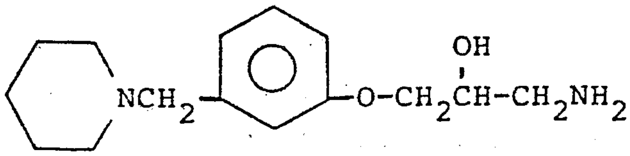

(II)

in an sich bekannter Weise

a) mit einem Thiadiazol-Derivat der Formel III

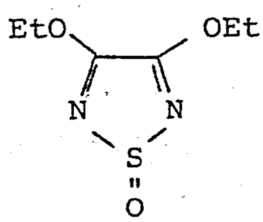

(III)

oder

b) mit einem Cyclobutendion-Derivat der Formel IV

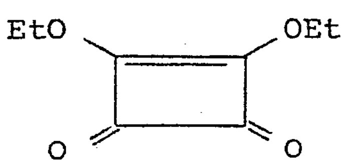

(IV)

umsetzt

und anschließend die erhaltene Verbindung mit einer alkoholischen, vorzugsweise ethanolischer, Ammoniaklösung zu den erfindungsgemäßen Verbindungen der allgemeinen Formel I umsetzt.

Die Umsetzung erfolgt bei einer Temperatur von Raumtemperatur bis Siedetemperatur des jeweiligen Lösungsmittels, vorzugsweise bei 25°C. Geeignete Lösungsmittel sind zum Beispiel Alkohole, wie Methanol, Ethanol oder Isopropanol, vorzugsweise Ethanol oder Ether, wie Dioxan oder Tetrahydrofuran.

Die Abtrennung der erhaltenen Verbindung der allgemeinen Formel I erfolgt in üblicher Weise, beispielsweise durch Chromatographie oder Kristallisation.

Die erhaltene Verbindung der allgemeinen Formel I kann in an sich bekannter Weise mit einer pharmakologisch annehmbaren Säure in ihr Salz umgewandelt werden.

Die erfindungsgemäßen Verbindungen, vorzugsweise in der Form eines Salzes, können zur Verabreichung in jeder beliebigen Weise formuliert werden. Die Erfindung umfaßt daher auch Arzneimittel, die mindestens eine erfindungsgemäße Verbindung zur Verwendung in der Human- oder Veterinärmedizin enthalten. Solche Arzneimittel können herkömmlicherweise unter Verwendung von einem oder mehreren pharmakologisch annehmbaren Trägern oder Verdünnungsmitteln hergestellt werden.

Die erfindungsgemäßen Verbindungen können daher für die orale, bukkale, topische, parenterale oder rektale Verabreichung formuliert werden, wobei die orale Verabreichung bevorzugt wird. Für die orale Verabreichung kann das Arzneimittel in Form von beispielsweise Tabletten, Kapseln, Pulvern, Lösungen, Sirups oder Suspensionen vorliegen, die unter Verwendung von annehmbaren Verdünnungsmitteln auf herkömmliche Weise hergestellt worden sind. Für die bukkale Verabreichung kann das Arzneimittel die Form von Tabletten oder Briefchen einnehmen, die in herkömmlicher Weise formuliert worden sind.

Die erfindungsgemäßen Verbindungen können für die parenterale Verabreichung durch Bolusinjektion oder kontinuierliche Infusion formuliert werden. Formulierungen zur Injektion können in Dosiseinheitsform als Ampullen oder in Mehrfachdosenbehältern mit zugesetztem Konservierungsmittel vorliegen.

Die Arzneimittel können solche Formen, wie Suspensionen, Lösungen oder Emulsionen in öligen oder wäßrigen Trägern, einnehmen, und sie können Formulierungshilfsmittel, wie Suspendierungs-, Stabilisierungs- und/oder Dispergierungsmittel, enthalten. Alternativ kann der Wirkstoff auch in Pulverform zur Rekonstitution mit einem geeigneten Träger, zum Beispiel sterilem, pyrogenfreiem Wasser, vor dem Gebrauch vorliegen.

Die erfindungsgemäßen Verbindungen können auch für rektale Zubereitungen, zum Beispiel Suppositorien oder Retentionseinläufe, formuliert werden, die zum Beispiel herkömmliche Suppositoriengrundlagen, wie Kakaobutter oder andere Glyceride, enthalten.

Zur topischen Anwendung können die erfindungsgemäßen Verbindungen als Salben, Cremes, Gels, Lotionen, Pulver oder Sprays in herkömmlicher Weise formuliert werden.

Für die orale Verabreichung ist eine geeignete Tagesdosis an erfindungsgemäßen Verbindungen 1 bis 4 Dosen bis ingesamt 5 mg bis 1 g/Tag, vorzugsweise 5 bis 250 mg/Tag, je nach Zustand des Patienten. Im Einzelfall kann es gegebenenfalls erforderlich sein, von den genannten Mengen abzuweichen, und zwar in Abhängigkeit vom individuellen Verhalten gegenüber dem Wirkstoff bzw. der Art seiner Formulierung und dem Zeitpunkt bzw. Intervall, zu welchem die Verabreichung erfolgt. So gibt es zum Beispiel Fälle, wo mit weniger als der oben genannten Mindestmenge ausgekommen werden kann, während in

anderen Fällen die genannte obere Grenze überschritten werden muß.

Die erfindungsgemäßen Verbindungen zeichnen sich gegenüber anerkannt guten Arzneimitteln der gleichen Wirkungsrichtung durch eine Verbesserung der pharmakologischen Aktivitäten aus. Dies ergibt sich aus den Ergebnissen der im folgenden dargestellten pharmakologischen Vergleichsuntersuchungen.

Eine anerkannte Methode zur Bestimmung $H_2$-antagonistischer Wirksamkeit ist die Ermittlung der $pA_2$-Werte in vitro am isolierten Meerschweinchenvorhof.

| | $pA_2$-Werte | |
|---|---|---|
| Cimetidin | 6,31 | Vergleich |
| Ranitidin | 6,81 | Vergleich |
| Beispiel 1 | 7,31 | |
| Beispiel 2 | 7,14 | |

Die Erfindung wird anhand der folgenden Beispiele erläutert.

## Beispiel 1

4-Amino-3-[2-hydroxy-3-[3-(1-piperidinylmethyl) phenoxy] propyl]-amino-1,2,5-thiadiazol-1-oxid

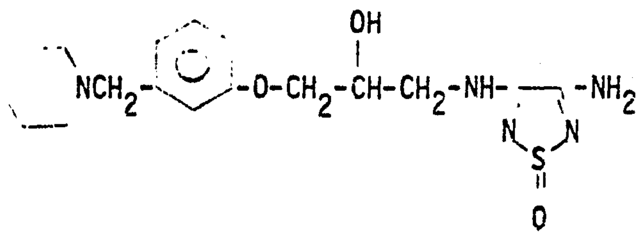

1.97 g (7mmol) 1-Amino-3-[3-(1-piperidinylmethyl) phenoxy]-2-propanol, gelöst in 50 ml Ethanol, werden mit 1.33 g (7 mmol) 3,4-Diethoxy-1,2,5-thiadiazol-1-oxid versetzt und 18 h bei 25 °C gerührt. Anschließend gibt man 15 ml einer 5n ethanolischen Ammoniaklösung zu und rührt nochmals 18 h bei 25 °C. Die Reaktionslösung wird im Vakuum eingeengt und der Rückstand säulenchromatographisch (Kieselgel/ Methanol) gereinigt.

Farblose Kristalle vom Schmelzpunkt 144 - 146 °C

Ausbeute: 850 mg (33 % d.Th.)

Rf = 0.57 (Methanol/$NH_3$/$H_2O$ 99 : 1 )

| | | | | | |
|---|---|---|---|---|---|
| $C_{17}H_{25}N_5O_3S$ (379,5) | Ber.: | C 53.81 | H 6.64 | N 18.46 | S 8.45 |
| | Gef.: | C 54.22 | H 6.82 | N 18.08 | S 8.66 |

$^1$H-NMR-Spektrum: ($d_6$-DMSO, TMS als interner Standard)

$\delta$ = 1.23 - 1.70 (m) ($-(CH_2)_3-$) 6 H,
2.17 - 2.47 (m) ( $(C\underline{H}_2)_2$) 4 H,
3.39 (s) (N-$C\underline{H}_2$) 2 H,
3.49 - 3.67 (m) ($-C\underline{H}_2-$) 2 H,
3.83 - 4.23 (-C$\underline{H}$, O-C$\underline{H}_2$) 3 H,
5.44 (m) (austauschbar mit $D_2O$) 1 H,
6.70 - 7.4 (m) (Aromaten-$\underline{H}$) 4 H,

8,0 (s, breit) (austauschbar mit $D_2O$) 2H,
8.25 (breit) (austauschbar mit $D_2O$) 1 H ppm.

Herstellung der Vorstufen

a) 2-(Oxiranylmethyl)-1H-isoindol-1,3-(2H)-dion

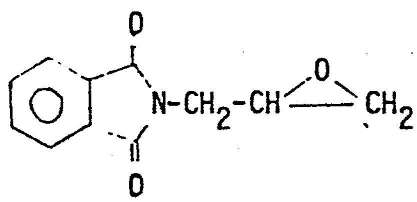

Zu einer Suspension von 55.6 g (0.3 mol) Kaliumphthalimid in 250 ml Dimethylformamid gibt man 41.1 g (0.3 mol) Epibromhydrin und erwärmt 8 h auf 100 °C. Nach Abkühlen der Reaktionslösung gießt man auf 300 ml Wasser, extrahiert mit Essigsäureethylester und engt das Lösungsmittel im Vakuum ein. Der Rückstand wird aus Ethanol/Essigsäureethylester/Petrolether umkristallisiert.

Farblose Kristalle vom Schmelzpunkt 90 - 95 °C

Ausbeute: 18.7 g (30.7 % d.Th.)

Rf = 0.4 ($Al_2O_3$; $CH_2Cl_2$)

b) 2-[2-Hydroxy-3-[3-(1-piperidinylmethyl) phenoxy] propyl -1H-isoindol-1,3-(2H)-dion

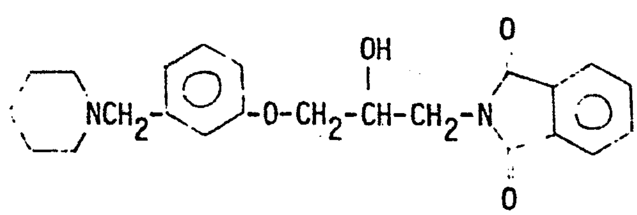

18,7 g (0,092 mol) 2-(Oxiranylmethyl)-1H-isoindol-1,3-(2H)-dion werden mit 17.5 g (0.092 mol ) 3-(1-Piperidinylmethyl)phenol vermischt und 10 Minuten auf 130 °C erhitzt. Nach dem Abkühlen löst man das Reaktionsgemisch in 200 ml Chloroform, wäscht die organische Phase nacheinander dreimal mit je 30 ml 1n-NaOH Lösung, 30 ml Wasser und 30 ml gesättigter wäßriger NaCl-Lösung. Nach dem Trocknen der organischen Phase über $Na_2SO_4$ engt man im Vakuum ein. Der Rückstand enthält als zähflüssiges Öl die Titelverbindung.

Ausbeute: 20.1 g (55 % d.Th.)

c) 1-Amino-3-[3-(1-piperidinylmethyl) phenoxy]-2-propanol

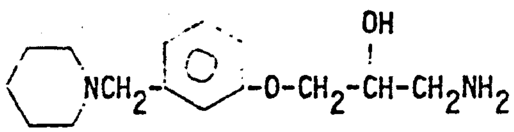

Eine Lösung aus 7.7 g (0.019 mol) 2-[2-Hydroxy-3-[3-(1-piperidinylmethyl) phenoxy]propyl]-1H-isoindol-1,3-(2H)-dion und 2.9 ml Hydrazinhydrat (80 %-ige wäßrige Lösung) in 80 ml Ethanol werden 3 h auf Rückflußtemperatur erhitzt. Nach dem Abkühlen der Reaktionslösung trennt man vom entstandenen Feststoff ab, engt die Lösung im Vakuum ein und löst den Rückstand in 80 ml Wasser. Zu der wäßrigen Lösung gibt man 7.2 ml conc. HCl, saugt vom ausgefallenen Feststoff ab, stellt die wäßrige Lösung mit 2n NaOH auf pH 12 ein und extrahiert mehrmals mit Essigsäureethylester. Das organische Lösungsmittel wird im Vakuum eingeengt und der Rückstand durch Destillation gereinigt. ($Kp_{0.008}$ = 140 - 160 °C). Nach Zugabe von Petrolether zum Destillat kristallisiert die Titelverbindung aus.

Farblose Kristalle vom Schmelzpunkt 72 - 74 °C

Ausbeute: 1.97 g (39 % d. Th.)

0132541

## Beispiel 2

2-Amino-1-[2-hydroxy-3-[3-(1-piperidinylmethyl)phenoxy]propyl]-amino-cyclo-but-1-en-3,4-dion

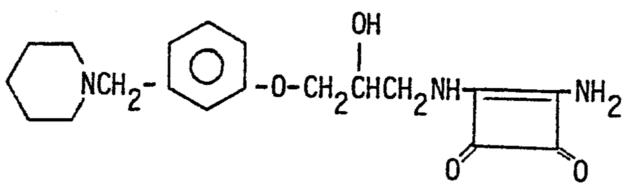

Die Herstellung erfolgt analog Beispiel 1 aus 5,28g(20 mmol)1-Amino-3-[3-(1-piperidinyl-methyl)phenoxy]-2-propanol und 3.4 g (20 mmol) 1,2-Diethoxycyclobut-1-en-3,4-dion.

Farblose Kristalle vom Schmelzpunkt 207 - 208 °C (zers.)

Ausbeute: 3.37 g (47 % d. Th.)

Rf = 0.18 ($CH_2Cl_2$/MeOH 8 : 2 )

$C_{19}H_{25}N_3O_4$ (359.4) Ber.: C 63,49 H 7.01 N 11.69

Gef.: C 63,44 H 7.06 N 11.68

$^1$H-NMR-Spektrum: (d$_6$-DMSO, TMS als interner Standard)

$\delta$ = 1.20 - 1.67 (m) (-($CH_2$)$_3$) 6 H,
2.17 - 2.47 (m) ( ($CH_2$)$_2$) 4 H,
3.40 (s) (N-$CH_2$) 2 H,
3.50 - 4.13 (m) (-$CH_2$-, -CH, O-$CH_2$, OH) 6 H,
6.73 - 7.40 (m) (Aromaten-H) 4 H,
7.50 (breit) 3 H (austauschbar mit $D_2O$) ppm.

LUDWIG HEUMANN & CO GMBH

8500 Nürnberg

---

Propan-2-ol-Derivate, Verfahren zu ihrer Herstellung und diese Verbindungen enthaltende Arzneimittel

---

## P A T E N T A N S P R Ü C H E

1. Propan-2-ol-Derivate der allgemeinen Formel I:

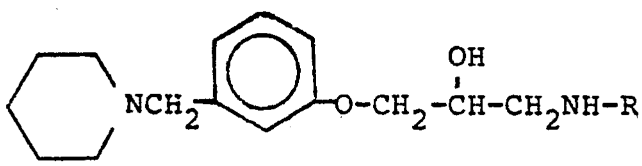

(I)

in der R für

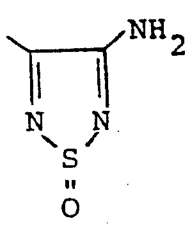

oder

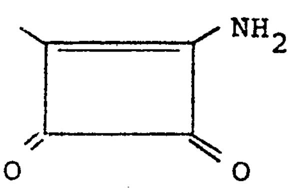

steht, und deren Salze mit pharmakologisch annehmbaren Säuren.

2. Verfahren zur Herstellung der Propan-2-ol-Derivate nach Anspruch 1, dadurch g e k e n n z e i c h n e t , daß man ein Propan-2-olamin der Formel II

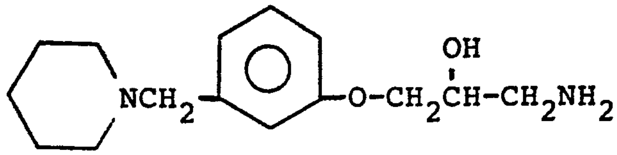 (II)

in an sich bekannter Weise mit einem Thiadiazol-Derivat der Formel III oder mit einem Cyclobutendion-Derivat der Formel IV

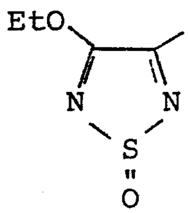 (III)

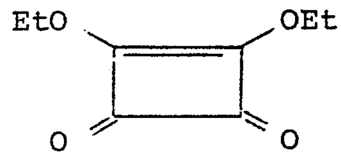 (IV)

zu einem Propan-2-ol-Derivat der Formeln V bzw. VI

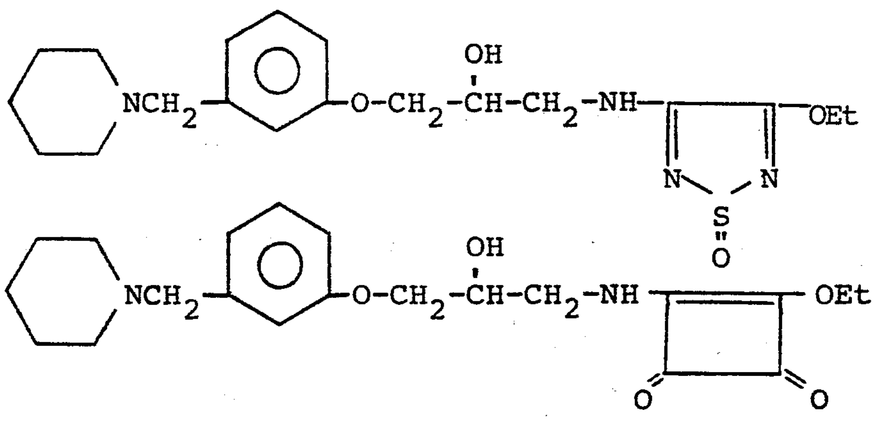 (V) (VI)

umsetzt und anschließend mit einer alkoholischen Ammoniaklösung zu einer Verbindung der allgemeinen Formel I umsetzt und gegebenenfalls die erhaltenen Verbindungen in ihr physiologisch annehmbares Salz umwandelt.

3. Arzneimittel, dadurch gekennzeichnet, daß es eine Verbindung nach Anspruch 1 und mindestens einen inerten, pharmazeutisch annehmbaren Träger oder ein inertes, pharmazeutisch annehmbares Verdünnungsmittel, gegegebenenfalls zusammen mit mindestens einem weiteren Wirkstoff, enthält.

Anspruch für Vertragsstaat: AT

## Patentanspruch

Verfahren zur Herstellung von Propan-2-ol-Derivaten der allgemeinen Formel I:

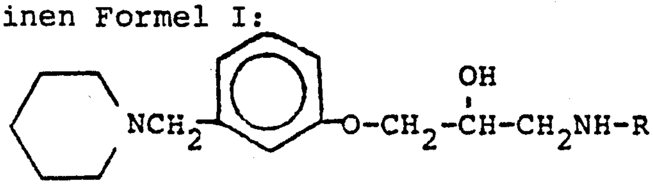

(I)

in der R für

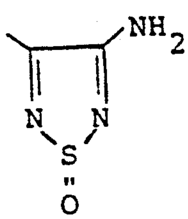

oder

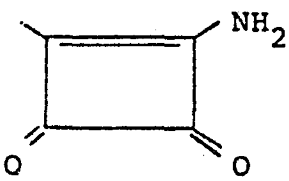

steht, und deren Salzen mit pharmakologisch annehmbaren Säuren, dadurch gekennzeichnet, daß man ein Propan-2-olamin der Formel II:

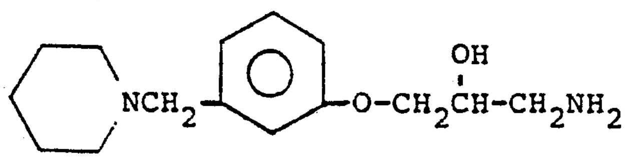

(II)

mit einem Thiadiazol-Derivat der Formel III oder mit einem Cyclobutendion-Derivat der Formel IV:

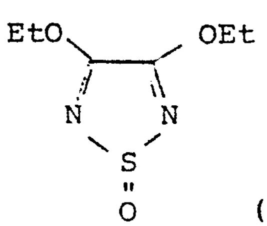

(III)

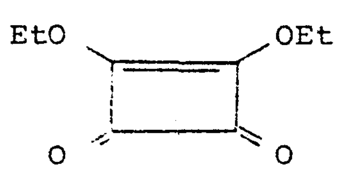

(IV)

zu einem Propan-2-ol-Derivat der Formeln V bzw. VI:

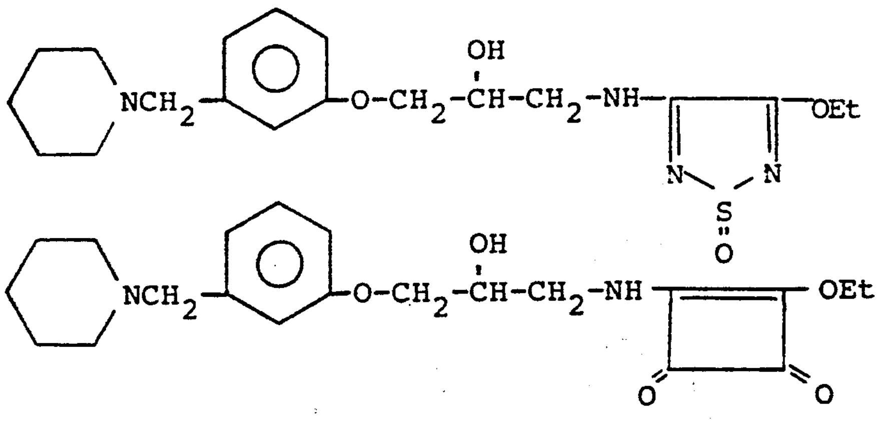

(V)

(VI)

umsetzt und anschließend mit einer alkoholischen Ammoniaklösung zu einer Verbindung der allgemeinen Formel I umsetzt und gegebenenfalls die erhaltenen Verbindungen in ihr physiologisch annehmbares Salz umwandelt.